# EUROPÄISCHE PATENTSCHRIFT

(11) **EP 3 231 466 B1**
(45) Veröffentlichungstag und Bekanntmachung des Hinweises auf die Patenterteilung: **14.08.2019**
(21) Anmeldenummer: 17165300.9
(22) Anmeldetag: 06.04.2017
(51) Int. Cl.: A61M 1/36

(54) **VERFAHREN ZUR ENTLEERUNG EINES GERÄTS ZUR EXTRAKORPORALEN BLUTBEHANDLUNG**
METHOD FOR EMPTYING A DEVICE FOR EXTRACORPOREAL BLOOD TREATMENT
PROCÉDÉ DESTINÉ À LA VIDANGE D'UN APPAREIL POUR LE TRAITEMENT SANGUIN EXTRACORPOREL

(30) Priorität: 15.04.2016 DE 102016107026
(43) Veröffentlichungstag der Anmeldung: 18.10.2017
(73) Patentinhaber: B. Braun Avitum AG, 34212 Melsungen (DE)
(72) Erfinder: RIEMENSCHNEIDER, Heiko, 34327 Wagenfurth (DE)
(74) Vertreter: Winter, Brandl, Fürniss, Hübner, Röss, Kaiser, Polte - Partnerschaft mbB

(56) Entgegenhaltungen:
- EP-B1- 1 161 271
- WO-A1-2008/028579
- US-A- 4 770 769
- US-A1- 2012 265 117

## Beschreibung

Die Erfindung betrifft ein Verfahren zur Entleerung eines Geräts zur extrakorporalen Blutbehandlung, und bezieht sich insbesondere auf ein Verfahren zur automatisierten und vollständigen Entleerung sowohl eines Schlauchsystems als auch einer Dialysatorvorrichtung für ein Gerät zur extrakorporalen Blutbehandlung nach Blutrückgabe über die Dialysatorvorrichtung.

WO 2008/028579 A1 offenbart ein Verfahren zur Entleerung der Blutschläuche an einem Dialysator. Dabei werden die arterielle Blutleitung und die venöse Blutleitung zunächst durch Konnektoren an den Leitungen zu einem Kreislauf kurzgeschlossen. Eine Substituatleitung wird dann von der Substituatquelle getrennt und anschließend wird über die Substituatpumpe Luft in den Kreislauf gefördert, wodurch die vorhandene Flüssigkeit in den Blutschläuchen in den Dialysator gefördert und über die Dialysemembran in die wasserseitige Kammer abgeführt wird, sodass der Kreislauf entleert wird.

EP 1 161 271 B1 offenbart ein Verfahren zum Entleeren eines extrakorporalen Blutkreislaufs an einer Vorrichtung zur extrakorporalen Blutbehandlung, bei der nach Therapieende zunächst die Enden der arteriellen Blutleitung und venösen Blutleitung kurzgeschlossen werden, sodass ein Kreislauf entsteht. Wasserseitig angeordnete Pumpen werden so gesteuert, dass der Druck in der wasserseitigen Kammer des Dialysators geringer ist als der Druck der blutseitigen Kammer. Während des Entleerungsvorgangs ist zumindest eine Blutpumpe in Betrieb, sodass die im kurzgeschlossenen Blutkreislauf vorhandene Flüssigkeit in Therapiefließrichtung in die blutseitige Kammer gefördert wird, dort durch das Druckgefälle in die wasserseitige Kammer übertritt und abgezogen wird.

Ein weiteres bekanntes Gerät für extrakorporale Blutbehandlung umfasst zumindest eine Behandlungseinheit, wie beispielsweise einen Dialysator bzw. eine Dialysatorvorrichtung, oder einen Filter, Ultrafilter oder Plasmafilter einer andersartigen Filtereinheit mit einer semipermeablen Membran, welche die Behandlungseinheit in zwei Kammern trennt. Ein extrakorporaler Blutkreislauf ermöglicht einen Fluss von aus einem Patienten entnommenem Blut durch die erste Kammer hindurch zurück zum Patienten. Gleichzeitig fließt gegenläufig dazu eine Dialysierflüssigkeit (Behandlungsflüssigkeit) durch einen geeignet ausgelegten Kreislauf über die zweite Kammer. Das bekannte Gerät beinhaltet darüber hinaus eine einlassseitig mit der zweiten Kammer verbundene Dialysierflüssigkeitsleitung für frische Dialysierflüssigkeit und eine auslassseitig mit der zweiten Kammer verbundene Dialysierflüssigkeitsleitung für verbrauchte Dialysierflüssigkeit.

Nach Therapieende und Blutrückgabe steht der Anwender des bekannten Geräts in der Regel vor der Problematik einer geeigneten Entleerung des (Fluid)Systems des Geräts. Bislang werden im Hinblick auf eine Entleerung lediglich eine möglicherweise während der Therapie verwendete Kartusche, bspw. einer Bikarbonat-Kartusche, sowie die Dialysatorvorrichtung (der Dialysator) auf der Wasserseite unterstützt. Die Blutseite dagegen verbleibt in der Regel gefüllt. Dies birgt Infektionsrisiken und erhöht in unnötiger Weise die Gewichtsmenge zu entsorgender Einmalartikel, wie des Dialysators und des Blutschlauchsystems. Außerdem gelangen selbst bei größter Sorgfalt des Anwenders regelmäßig Blutreste und/oder Restbestandteile von frischer und/oder verbrauchter Dialysierflüssigkeit auf die umliegende Bodenfläche und/oder auf das Gerät bzw. die Maschine selbst.

Bekannte Verfahren und/oder Vorrichtungen beinhalten allgemein ein Durchspülen des Schlauchsystems mit einer Flüssigkeit, wie bspw. Kochsalzlösung oder Dialysierflüssigkeit, um restliches Blut zu entfernen, und Abführen der Spülflüssigkeit über einen dedizierten Auslass.

In der Regel erhält ein Anwender eine Beschreibung und Schulung dahingehend, wie ein Gerät oder Maschine zur extrakorporalen Blutbehandlung nach der Therapie wieder abzurüsten ist. Hierbei werden jedoch lediglich die Wasserseite des Systems und die Kartusche entleert. Wie im Hinblick auf blutseitige Restmengen im Blutschlauchsystem vorzugehen ist, wird dagegen nicht betrachtet. Blutseitig jährlich anfallende und kostenintensiv als Sondermüll zu entsorgende Rest- bzw. Müllmengen sind jedoch erheblich.

Es besteht daher Bedarf an einer Lösung, welche den Anwender wie bei der (wasserseitigen) Entleerung des Systems und der Kartusche auch bei der (blutseitigen) Entleerung des Schlauchsystems einschließlich des Dialysators ebenfalls vollautomatisch unterstützt.

Der Erfindung liegt daher als eine Aufgabe zugrunde, ein Verfahren zur Entleerung eines Geräts zur extrakorporalen Blutbehandlung bereitzustellen, welches einen Anwender des Geräts während eines Entleerungsbetriebsablaufs nach Therapieende durch einen automatisierten und einfach und sicher handhabbaren Prozess unterstützt.

Außerdem soll die Erfindung einen einfachen und weitgehend vollautomatischen Prozess zur vollständigen Entleerung des Schlauchsystems einschließlich des Dialysators nach der Blutrückgabe über den Dialysator schaffen.

Darüber hinaus soll die Erfindung Infektionsgefahren minimieren und kostensparend anfallende Müllmengen verringern.

Diese Aufgabe wird erfindungsgemäß durch ein Verfahren zur Entleerung eines Geräts zur extrakorporalen Blutbehandlung mit den Merkmalen des Anspruchs 1 gelöst. Vorteilhafte Weiterbildungen der Erfindung sind Gegenstand der beigefügten Unteransprüche.

Der Erfindung liegt als eine allgemeine Idee zugrunde, auch die Blutseite inklusive vorhandener Restmengen im Dialysator unter Nutzung der Dialysatormembran und mit Unterstützung einer Pegelregulierung, alternativ einer manuell herbeigeführten Druckunterstützung, automatisch zu entleeren, d.h. einen Prozess bereitzustellen, mittels welchem an einem Gerät zur extrakorporalen Blutbehandlung software- und/oder hardware-unterstützt Restmengen in dessen Schlauchsystem unter Nutzung der Dialysatormembran mit Unterstützung der Pegel- bzw. Level-Regulierung und/oder manueller Druckunterstützung der Drucksensoren und der Blutpumpe zur vollautomatischen Entleerung der Blutseite einschließlich aller Kammern und des Dialysators nach Therapieende ausspülbar sind.

Nachdem der Anwender zum Therapieende das Blut an einen Patienten zurückgegeben hat, wendet er sich wieder dem Gerät zu. Beispielsweise hat er dann geräteseitig eine durch Tastendruck, Bildschirmberührung oder dergleichen aktivierbare Option "System entleeren", welches gleichbedeutend ist mit einer Anweisung an den Operator, die arteriellen und venösen Anschlüsse kurzzuschließen und den entsprechenden Vorgang zu bestätigen. Nach Aktivierung dieser Option wird das Gerät über eine Level- bzw. Pegelregulierung Anschluss für Anschluss mit Druck beaufschlagt, läuft die Blutpumpe mit einer vorbestimmten Fördermenge, beispielsweise 100 ml/min, wird auf der Dialysierflüssigkeits- oder auch Wasserseite ein Unterdruck aufgebaut und werden alle Restmengen an Flüssigkeit über den Dialysator bzw. über dessen Membran von der Blutseite zur Wasserseite hin entzogen. Nach wenigen Minuten, beispielsweise 3 Minuten, ist das gesamte System einschließlich des Dialysators entleert, und kann es der Anwender wie gewohnt abnehmen bzw. abrüsten und das Gerät wieder für eine nachfolgende Behandlung vorbereiten.

Damit einhergehend ist eine signifikante Senkung der Betriebskosten und Verringerung anfallender Sondermüllmengen erzielbar. Durch das Entleeren auch der Blutseite einer Dialysemaschine sind weitere 200 bis 350 ml von als Sondermüll zu entsorgender Restmenge pro Behandlung vermeidbar. Pro Dialysemaschine liegt damit eine jährlich anfallende Sondermüllmenge in einem Bereich von 0,2 bis 0,35 t. Bei angenommenen Entsorgungskosten für Sondermüll aus Dialysemaschinen und Dialysebehandlungen von 350 Euro pro Tonne Sondermüll ist über die Anzahl der sich insgesamt in Betrieb befindenden Maschinen das Kostensenkungspotenzial erheblich.

Im Einzelnen wird die Aufgabe gelöst durch ein Verfahren zum Entleeren eines Geräts zur extrakorporalen Blutbehandlung, wobei das Gerät zumindest einen arteriellen Anschluss, einen venösen Anschluss, eine Dialysatorvorrichtung, eine Blutpumpe, eine Luftdetektoreinrichtung, eine Pegel-Regulierungseinrichtung und eine venöse Expansionskammer beinhaltet, und wobei das Verfahren die Schritte beinhaltet: Verbinden zumindest des arteriellen Anschlusses und des venösen Anschlusses des Geräts; Erzeugen eines wasserseitigen Unterdrucks; Entleeren der fluidführenden Leitungen des Geräts über die Membran der Dialysatorvorrichtung in einer ersten Richtung zum Dialysator hin, wobei während des Entleerens in der ersten Richtung die Blutpumpe in Betrieb gehalten wird; und Entleeren der fluidführenden Leitungen des Geräts über die Membran der Dialysatorvorrichtung in einer zweiten Richtung zum Dialysator hin, die der ersten Richtung entgegengesetzt ist, wobei während des Entleerens in der zweiten Richtung die Blutpumpe angehalten ist und während des Entleerens in der ersten Richtung und während des Entleerens in der zweiten Richtung eine Pegel-Regulierungseinrichtung in Betrieb gehalten wird.

Als Pegel-Regulierungseinrichtung wird eine Pegel-Regulierungspumpe verwendet, die dazu konfiguriert ist, eine Pegelregulierung zumindest in der venösen Expansionskammer bereitzustellen.

Während des Entleerens in der ersten Richtung und während des Entleerens in der zweiten Richtung sind arterielle und/oder venöse Fluidabgänge sowie ein Fluidabgang aus der venösen Expansionskammer manuell druckbeaufschlagend unterstützbar.

Bevorzugt erfolgt eine manuelle Druckbeaufschlagung durch Öffnen von Drucksensoranschlüssen und/oder Abklemmen zumindest eines Zugangs.

Bevorzugt wird ausgehend von einem Zeitpunkt, zu dem in der Luftdetektoreinrichtung ein Auftreten von Luft erfasst wird, eine Anzahl von Kopfumdrehungen der Blutpumpe gezählt, wobei einer Kopfumdrehung in Abhängigkeit von einer Förderkonstanten ein vorbestimmtes Fördervolumen pro einer Umdrehung zugeordnet ist.

Bevorzugt wird ein Abschaltzeitpunkt der Blutpumpe auf der Grundlage einer voreingestellten Ausspülmenge und der gezählten Anzahl von Kopfumdrehungen der Blutpumpe ermittelt, wobei zu dem ermittelten Abschaltzeitpunkt der Betrieb der Blutpumpe angehalten wird und ein Wechsel von der Entleerung in der ersten Richtung auf die Entleerung in der zweiten Richtung erfolgt.

Bevorzugt wird während der Entleerung in einer zweiten Richtung nach einer vorbestimmten Zeitspanne ein Unterdruck auch auf der Blutseite der Dialysatorvorrichtung aufgebaut.

Vorteilhaft ist der blutseitige Unterdruck an dem Anschluss eines venösen Druckaufnehmers messbar.

Bevorzugt werden nach Erfassen eines vorbestimmten blutseitigen Unterdrucks die fluidführenden Leitungen des Geräts wasserseitig abgeschlossen, wird eine Pumpeinrichtung in einem Ablauf für verbrauchte Dialysierflüssigkeit angehalten, und wird ein Anwender über das Ende der blutseitigen Entleerung des Geräts benachrichtigt.

Bevorzugt wird der Transmembrandruck berechnet und der Entleerungsbetriebsablauf unterbrochen, wenn der berechnete Transmembrandruck einen vorbestimmten Grenzwert überschreitet.

Bevorzugt wird die Membran der Dialysatorvorrichtung mittels einer Blutleckageerfassungseinrichtung im Ablauf für verbrauchte Dialysierflüssigkeit auf das Auftreten einer Ruptur hin überwacht.

Die Erfindung wird nachstehend anhand eines bevorzugten Ausführungsbeispiels unter Bezugnahme auf die beigefügte Zeichnung näher beschrieben. Es zeigen:
Fig. 1 schematisch einen ersten Abschnitt eines Ablaufs bei dem Verfahren zur Entleerung eines Geräts zur extrakorporalen Blutbehandlung gemäß einem Ausführungsbeispiel;
Fig. 2 schematisch einen zweiten Abschnitt eines Ablaufs bei dem Verfahren zur Entleerung eines Geräts zur extrakorporalen Blutbehandlung gemäß dem Ausführungsbeispiel; und
Fig. 3 schematisch einen dritten Abschnitt eines Ablaufs bei dem Verfahren zur Entleerung eines Geräts zur extrakorporalen Blutbehandlung gemäß dem Ausführungsbeispiel.

In der nachfolgenden Figurenbeschreibung können in einzelnen Figuren gleiche oder gleichwirkende Elemente und/oder Komponenten gleich und/oder mit denselben Bezugszeichen bezeichnet und zweckmäßig nicht redundant beschrieben sein. In Fällen, in welchen ein nachfolgendes Ausführungsbeispiel funktionell zumindest einem vorangehenden entspricht, d.h. entsprechende Funktionen, Anordnungen und/oder Verfahrens- oder Betriebsabläufe gleichermaßen umfasst sind, wird zweckmäßig nur auf Unterschiede eingegangen.

Ein Gerät zur extrakorporalen Blutbehandlung, wie beispielsweise eine Dialysemaschine zur Reinigung von Blut eines Patienten, wenn z.B. dessen Nierenfunktion eingeschränkt oder eingestellt ist, weist einen Dialysator auf, der einerseits von dem zu reinigenden Blut des Patienten und andererseits von einer Dialysierflüssigkeit oder Dialyselösung, vorzugsweise gemäß dem Gegenstromprinzip, durchströmt wird, wobei gewisse gelöste Substanzen (z.B. Harnstoff) aus dem Blut in die Dialysierflüssigkeit übergehen.

Systematik, Aufbau, Komponenten und Funktionsweise des vorgenannten Geräts zur extrakorporalen Blutbehandlung, das insbesondere ein Dialysegerät oder eine Dialysemaschine sein kann, sind an sich grundlegend bekannt und werden daher diesbezüglich hierin einbezogen und nicht weiter beschrieben.

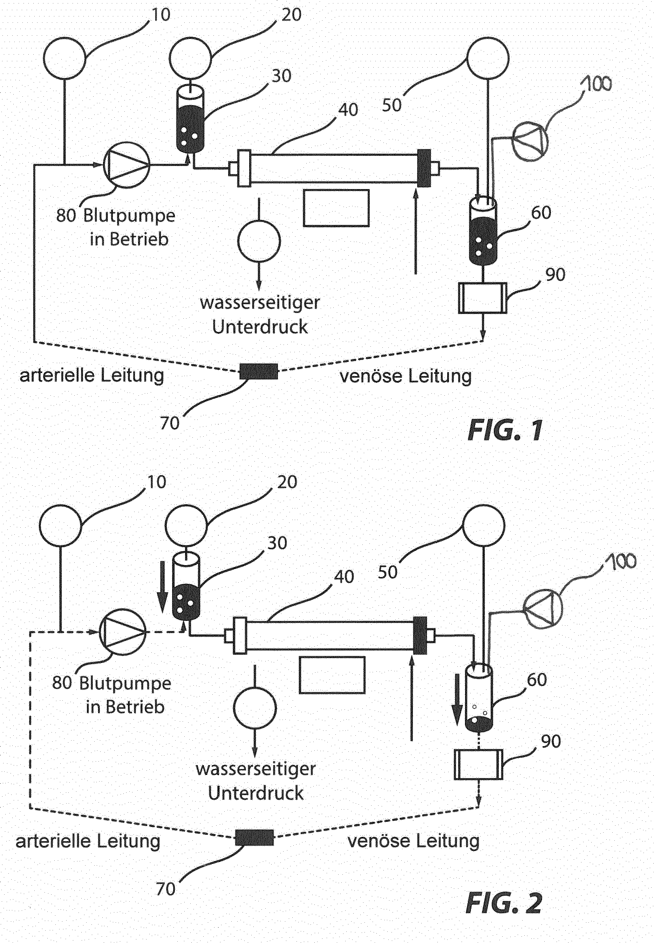

Die Fig. 1 bis 3 stellen jeweils schematisch einen ersten, einen zweiten und einen dritten Abschnitt eines Ablaufs bei dem Verfahren zur Entleerung eines Geräts zur extrakorporalen Blutbehandlung gemäß einem Ausführungsbeispiel dar. Es wird angemerkt, dass sich die Fig. 1 bis 3 auf die Blutseite des Geräts beziehen, d.h. die schematisch dargestellten Fluidleitungen blutführend sind.

Gemäß den Fig. 1 bis 3 beinhaltet ein mit dem Verfahren zur Entleerung eines fluidführenden Systems eines Geräts zur extrakorporalen Blutbehandlung, wie beispielsweise eines Dialysegeräts oder einer Dialysemaschine, zu entleerendes System fluidführende Verbindungsleitungen bzw. Schlauchleitungen, die in jeweils zumindest einen arteriellen und zumindest einen venösen Anschluss münden.

In einer arteriellen Fluidleitung sind ein Dialysator 40 mit einer Dialysatormembran (nicht gezeigt), ein arterieller Druckaufnehmer 10, eine Blutpumpe 80, eine erste (arterielle) Expansionskammer (Blasenkammer, Blasenfänger) 30 vor dem Dialysator 40, ein Druckaufnehmer 20 an der ersten Expansionskammer 30, eine zweite (venöse) Expansionskammer (Blasenkammer, Blasenfänger) 60 nach dem Dialysator 40, ein venöser Druckaufnehmer 50 an der zweiten Expansionskammer 60 und ein Luftdetektor 90 angeordnet. Eine Verbindervorrichtung (Adapter) 70 ist vorgesehen und dazu angeordnet, einen arteriellen Anschluss und einen venösen Anschluss des Fluid- oder Schlauchsystems des Geräts fluiddurchlässig zu verbinden bzw. kurzzuschließen.

Nachstehend wird der Ablauf bei dem Verfahren zur Entleerung eines Geräts zur extrakorporalen Blutbehandlung gemäß dem vorliegenden Ausführungsbeispiel näher beschrieben.

Nachdem der Anwender des Geräts zur extrakorporalen Blutbehandlung zum Therapieende das Blut an einen Patienten zurückgegeben hat, wendet er sich wieder dem Gerät zu. Soweit vorhanden, kann zu diesem Zeitpunkt eine (nicht gezeigte) (Bikarbonat-)Kartusche bereits entleert worden sein. Nach Betätigen einer Auswahleinrichtung, beispielsweise einer Taste, eines Schalters oder eines berührungsempfindlichen Felds auf einer bildschirmgestützten Benutzeroberfläche, wird das Gerät in einen Betriebsablauf "System entleeren" versetzt.

Der Anwender schließt nun manuell die arteriellen und venösen Anschlüsse des Blutschlausystems mittels der Verbindervorrichtung 70, die beispielsweise ein (Schlauch- oder Anschluss-) Adapter sein kann, zusammen, d.h. verbindet diese fluidführend, und bestätigt gegenüber dem Gerät die Entleerung mittels der Auswahleinrichtung (der Taste "System entleeren"). Es wird angemerkt, dass die beiden Anschlüsse des Blutschlauchsystems bis zum Ende des Entleerungsvorgangs mittels der Verbindervorrichtung 70 verbunden bleiben.

Geräteseitig wird nun zunächst unter Verwendung einer (nicht gezeigten) Dialysierflüssigkeitspumpe, die vorzugsweise im Ablauf der verbrauchten Dialysierflüssigkeit angeordnet ist, und entsprechender Ventilbeschaltung ein (wasserseitiger) Unterdruck auf der Dialysierflüssigkeitsseite aufgebaut, vorzugsweise z.B. etwa 500 mm Hg, messbar durch einen geeignet angeordneten Sensor, beispielsweise einen Druck-Sensor. Die Blutpumpe 80 beginnt mit einer Drehzahl von beispielsweise 50 1/min zu arbeiten. Eine Pegel- oder Level-Regulierungspumpe 100 drückt nun den Pegel in der zweiten (venösen) Expansionskammer 60 nach unten. Der Pegel in dieser zweiten Expansionskammer 60 senkt sich dadurch entsprechend, bis Luft (Luftblasen) an dem Luftdetektor 90 erfasst wird. Bei erstmaliger Erfassung von Luft bzw. Luftblasen an dem Luftdetektor 90 wird nun die Anzahl von Kopfumdrehungen an der Blutpumpe 80 (Förderkonstante) mitgerechnet bzw. gezählt (beispielsweise werden jeweils 12,3 ml Fluidförderung pro Umdrehung der Blutpumpe 80 aufaddiert).

Die in dem kurzgeschlossenen Blutschlauchsystem befindliche Flüssigkeitsmenge wird zum einen aufgrund des Pumpendrucks der Blutpumpe 80 und zum anderen aufgrund des wasserseitig anliegenden Unterdrucks von der venösen Leitung über die Verbindervorrichtung 70, die arterielle Leitung, die erste Kammer des Dialysators 40, die Membran und die zweite Kammer des Dialysators 40 zur Wasserseite entzogen. Dabei fällt auch der Pegel in der ersten (arteriellen) Expansionskammer 30 (Fig. 2). Der Betrieb der Blutpumpe 80 wird so lange fortgesetzt, d.h. die Blutpumpe 80 wird so lange weiter in Betrieb gehalten, bis das System bis zu einem Einlauf des Dialysators 40 mit Luft gefüllt bzw. von Ausspülresten entleert ist.

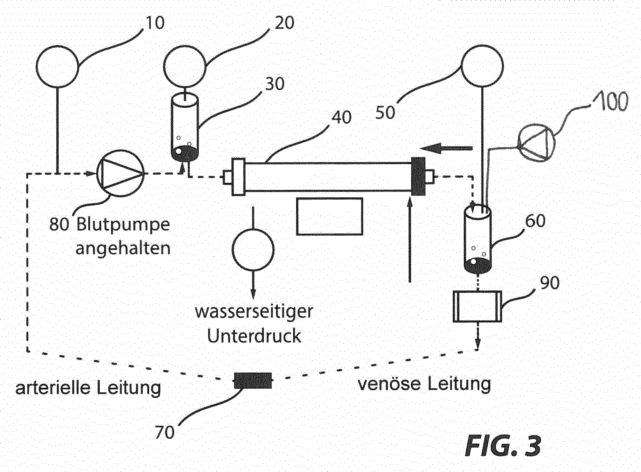

Entsprechende Ausspülmengen sind schlauchsystemabhängig und durch den Anwender einstellbar. Das Gerät errechnet bei bekannter Förderkonstante, beispielsweise 12,3 ml/Kopfumdrehung der Blutpumpe 80, einen Abschaltzeitpunkt der Blutpumpe 80 aus der jeweils eingestellten Ausspülmenge und der Anzahl der Kopfumdrehungen der Blutpumpe 80. Bei Erreichen der eingestellten Ausspülmenge wird der Betrieb der Blutpumpe 80 angehalten, d.h. die Blutpumpe stoppt (Fig. 3).

Der venöse Druckaufnehmer 50 wird jedoch aufgrund des fortgesetzten Betriebs der Pegel- oder Level-Regulierungspumpe 100 weiter mit Druck beaufschlagt, so dass das System beginnt, sich gegen die normale Flussrichtung (erste Richtung) zurück vom venösen Druckaufnehmer 50 zum Dialysator 40 (zweite Richtung, die der ersten Richtung entgegengesetzt ist) zu entleeren.

Nach kurzer Zeit, im vorliegenden Ausführungsbeispiel etwa einer Minute, wird der Betrieb auch der Pegel- bzw.- Level-Regulierungspumpe 100 angehalten. Wenige Sekunden später, im vorliegenden Ausführungsbeispiel nach etwa 10 Sekunden, ist auch auf der Blutseite ein Unterdruck aufgebaut. Dieser Unterdruck auf der Blutseite ist beispielsweise über den venösen Druckaufnehmer 50 messbar.

Nun schließen (nicht gezeigte) Ventile die Wasserseite des Geräts ab, wird die Dialysierflüssigkeitspumpe angehalten, und wird ein Hinweis bzw. eine Meldung an den Anwender ausgegeben, dass die Systementleerung blutseitig abgeschlossen ist.

Sodann koppelt der Anwender die wasserseitige (beispielsweise blaue) Kupplung vom Dialysator 40 ab und bestätigt dies. Dadurch leert sich sodann auch die Wasserseite des Dialysators 40. Danach ist das gesamte System entleert und kann von dem Anwender abgenommen werden. Nachfolgend kann das Gerät in der vorgesehenen Weise für eine nächste Behandlung vorbereitet werden.

Als ein die Sicherheit erhöhendes Merkmal kann zusätzlich der Transmembrandruck (TMP) an der Membran des Dialysators 40 berechnet werden und der Entleerungsablauf unterbrochen werden, wenn dieser Druck zu hoch wird und einen vorbestimmten Grenzwert überschreitet.

Wie vorstehend beschrieben wurde, werden bei einem Verfahren zum Entleeren eines Geräts zur extrakorporalen Blutbehandlung mit zumindest einem arteriellen und einem venösen Anschluss eines Blutschlauchsystems, einer Dialysatorvorrichtung, einer Blutpumpe, einer Luftdetektoreinrichtung, einer Pegel-Regulierungspumpe und einer venösen Expansionskammer zumindest der arterielle Anschlusses und der venöse Anschluss des Blutschlauchsystems verbunden. Sodann wird ein wasserseitiger Unterdruck erzeugt und werden die fluidführenden Leitungen des Blutschlauchsystems über die Dialysatormembran zunächst in einer ersten Richtung zum Dialysator hin, wobei während des Entleerens in der ersten Richtung die Blutpumpe in Betrieb gehalten wird, und sodann ebenfalls über die Dialysatormembran in einer zweiten Richtung zum Dialyator hin, die der ersten Richtung entgegengesetzt ist, wobei während des Entleerens in der zweiten Richtung die Blutpumpe angehalten ist, entleert.

Während des Entleerens in der ersten Richtung zum Dialysator hin und während des Entleerens in der zweiten Richtung zum Dialysator hin kann eine Pegel-Regulierungseinrichtung 100 in Betrieb gehalten werden, oder können alternativ arterielle und/oder venöse Fluidabgänge sowie ein Fluidabgang aus der venösen Expansionskammer manuell druckbeaufschlagend unterstützt werden. Ausgehend von einem Zeitpunkt, zu dem in der Luftdetektoreinrichtung ein Auftreten von Luft erfasst wird, wird eine Anzahl von Kopfumdrehungen der Blutpumpe gezählt, wobei einer Kopfumdrehung in Abhängigkeit von einer Förderkonstanten ein vorbestimmtes Fördervolumen pro einer Umdrehung zugeordnet ist. Ein Abschaltzeitpunkt der Blutpumpe wird auf der Grundlage einer voreingestelltenAusspülmenge und der gezählten Anzahl von Kopfumdrehungen der Blutpumpe ermittelt, wobei zu dem ermittelten Abschaltzeitpunkt der Betrieb der Blutpumpe angehalten wird und ein Wechsel von der Entleerung in der ersten Richtung zum Dialysator hin auf die Entleerung in der zweiten Richtung zum Dialysator hin erfolgt. Während der Entleerung in einer zweiten Richtung wird nach einer vorbestimmten Zeitspanne ein Unterdruck auch auf der Blutseite der Dialysatorvorrichtung aufgebaut, der an dem Anschluss eines venösen Druckaufnehmers messbar ist.

Nach Erfassen eines vorbestimmten blutseitigen Unterdrucks werden die fluidführenden Leitungen des Geräts wasserseitig abgeschlossen, wird eine Pumpeinrichtung im Ablauf für verbrauchte Dialysierflüssigkeit angehalten, und wird ein Anwender über das Ende der blutseitigen Entleerung des Geräts benachrichtigt.

Absichernd kann der Transmembrandruck berechnet und der Entleerungsbetriebsablauf unterbrochen werden, wenn der berechnete Transmembrandruck einen vorbestimmten Grenzwert überschreitet. Ferner kann die Membran der Dialysatorvorrichtung mittels einer Blutleckageerfassungseinrichtung im Ablauf für verbrauchte Dialysierflüssigkeit auf das Auftreten einer Ruptur hin überwacht werden.

Es versteht sich, dass die Erfindung nicht auf das beschriebene Ausführungsbeispiele und dessen Modifikationen beschränkt ist, sondern dass sich innerhalb des durch die nachstehenden Ansprüche definierten Schutzumfangs für den Fachmann gleichwohl naheliegend Kombinationen von zumindest Teilen dieser Ausführungsbeispiele, Modifikationen und Äquivalente ergeben können.

In einer Modifikation können beispielsweise auch Blutschlauchsysteme an Geräten ohne Levelregulierung entleert werden. In diesem Fall kann vorgesehen sein, dass ein Anwender die arteriellen und venösen Abgänge und die Expansionskammer(n) durch Öffnen der Drucksensoranschlüsse und durch Abklemmen der Zugänge unterstützt.

Ferner kann vorgesehen sein, zur Erhöhung der Sicherheit den Entleerungsprozess durch die Druckaufnehmer (wasserseitig und/oder blutseitig) zu überwachen. Hierbei kann ein zulässiger Grenzbereich zur Überwachung der Dialysatormembran (Membranschutz gegen Durchbruch bei zu hohem Druck) und für einen sicherheitsbedingten Abbruch der Entleerung beispielsweise aus der Therapie übernommen werden. Für den Fall einer auftretenden Ruptur der Dialysatormembran kann vorgesehen sein, dass ein im Ablauf für verbrauchte Dialysierflüssigkeit angeordneter Blutleckagedetektor als eine Blutleckageerfassungseinrichtung diese erkennt und den Anwender darüber informiert.

## Patentansprüche

1. Verfahren zum Entleeren eines Geräts zur extrakorporalen Blutbehandlung, wobei das Gerät einen Dialysator (40), der mittels einer Membran in eine erste Kammer und eine zweiten Kammer geteilt ist, eine mit einem Bluteingang der ersten Kammer verbundene arterielle Leitung, eine mit einem Blutausgang der ersten Kammer verbundene venöse Leitung, eine mit einem Dialysierflüssigkeitseingang der zweiten Kammer verbundene Dialysierflüssigkeitsleitung für frische Dialysierflüssigkeit und eine mit einem Dialysierflüssigkeitsausgang der zweiten Kammer verbundene Dialysierflüssigkeitsleitung für verbrauchte Dialysierflüssigkeit, eine in der arteriellen Leitung angeordnete Blutpumpe (80) und eine in der venösen Leitung angeordnete venöse Expansionskammer (60) beinhaltet, wobei
das Verfahren die Schritte aufweist:
a) Verbinden eines patientenseitigen Anschlusses der arteriellen Leitung mit einem patientenseitigen Anschluss der venösen Leitung;
b) Erzeugen eines Unterdrucks in der zweiten Kammer;
c) Betreiben der Blutpumpe (80) in eine erste Richtung und Entleeren der arteriellen und venösen Leitungen in die erste Richtung über die Membran und die zweite Kammer, **dadurch gekennzeichnet, dass**
das Verfahren weiter den Schritt
d) Anhalten der Blutpumpe (80) und Entleeren der arteriellen und venösen Leitungen in einer der ersten Richtung entgegengesetzten zweiten Richtung über die Membran und die zweite Kammer aufweist;
das Gerät weiter zumindest eine Pegel-Regulierungspumpe beinhaltet, die dazu konfiguriert ist, eine Pegelregulierung zumindest in der venösen Expansionskammer (60) bereitzustellen, wobei während des Entleerens in der ersten Richtung und während des Entleerens in der zweiten Richtung die Pegel-Regulierungspumpe in Betrieb gehalten wird, oder während des Entleerens in der ersten Richtung und während des Entleerens in der zweiten Richtung die mit dem Bluteingang der ersten Kammer verbundene arterielle Leitung und/oder die mit dem Blutausgang der ersten Kammer verbundene venöse Leitung sowie die venöse Expansionskammer (60) manuell druckbeaufschlagend unterstützbar sind; und
das Gerät weiter eine der venösen Expansionskammer (60) nachgeschaltete Luftdetektoreinrichtung (90) beinhaltet.

2. Verfahren nach Anspruch 1, bei dem die manuelle Druckbeaufschlagung durch Öffnen von Drucksensoranschlüssen und/oder Abklemmen des jeweils auf der gegenüberliegenden Seite des Dialysators (40) befindlichen Bluteingangs oder Blutausgangs erfolgt.

3. Verfahren nach einem der vorangehenden Ansprüche, bei dem ausgehend von einem Zeitpunkt, zu dem in der Luftdetektoreinrichtung (90) ein Auftreten von Luft erfasst wird, eine Anzahl von Kopfumdrehungen der Blutpumpe (80) gezählt wird, wobei einer Kopfumdrehung in Abhängigkeit von einer Förderkonstanten ein vorbestimmtes Fördervolumen pro einer Umdrehung zugeordnet ist.

4. Verfahren nach Anspruch 3, bei dem ein Abschaltzeitpunkt der Blutpumpe (80) auf der Grundlage einer vorbestimmten Ausspülmenge und der gezählten Anzahl von Kopfumdrehungen der Blutpumpe (80) ermittelt wird, wobei zu dem ermittelten Abschaltzeitpunkt der Betrieb der Blutpumpe (80) angehalten wird und ein Wechsel von der Entleerung in der ersten Richtung auf die Entleerung in der zweiten Richtung erfolgt.

5. Verfahren nach einem der vorangehenden Ansprüche, bei dem während der Entleerung in einer zweiten Richtung nach einer vorbestimmten Zeitspanne ein Unterdruck auch auf der Blutseite des Dialysators (40) aufgebaut wird.

6. Verfahren nach Anspruch 5, bei dem der blutseitige Unterdruck an dem Anschluss eines venösen Druckaufnehmers (50) messbar ist.

7. Verfahren nach Anspruch 6, bei dem nach Erfassen eines vorbestimmten blutseitigen Unterdrucks die fluidführenden Leitungen des Geräts wasserseitig abgeschlossen werden, eine Pumpeinrichtung in einem Ablauf für verbrauchte Dialysierflüssigkeit angehalten wird, und ein Anwender über das Ende der blutseitigen Entleerung des Geräts benachrichtigt wird.

8. Verfahren nach einem der vorangehenden Ansprüche, bei dem der Transmembrandruck berechnet und der Entleerungsbetriebsablauf unterbrochen wird, wenn der berechnete Transmembrandruck einen vorbestimmten Grenzwert überschreitet.

9. Verfahren nach einem der vorangehenden Ansprüche, bei dem die Membran des Dialysators (40) mittels einer Blutleckageerfassungseinrichtung im Ablauf für verbrauchte Dialysierflüssigkeit auf das Auftreten einer Ruptur hin überwacht wird.

## Claims

1. A method of draining a device for extracorporeal blood treatment, the device comprising a dialyser (40) divided into a first chamber and a second chamber by means of a membrane, an arterial line connected to a blood inlet of the first chamber, a venous line connected to a blood outlet of the first chamber, a dialysis fluid line for fresh dialysis fluid connected to a dialysis fluid inlet of the second chamber and a dialysis fluid line for used dialysis fluid connected to a dialysis fluid outlet of the second chamber, a blood pump (80) disposed in the arterial line, and a venous expansion chamber (60) disposed in the venous line, wherein the method comprises the steps of:
a. connecting a patient-side port of the arterial line to a patient-side port of the venous line;
b. generating negative pressure in the second chamber;
c. operating the blood pump (80) in a first direction and draining the arterial and venous lines in the first direction via the membrane and the second chamber, **characterized in that**
the method further comprises the step of
d. stopping the blood pump (80) and draining the arterial and venous lines in a second direction opposed to the first direction via the membrane and the second chamber;
the device further includes at least one level control pump which is configured to provide level control at least in the venous expansion chamber (60), wherein during draining in the first direction and during draining in the second direction the level control pump is kept in operation, or during draining in the first direction and during draining in the second direction the arterial line connected to the blood inlet of the first chamber and/or the venous line connected to the blood outlet of the first chamber as well as the venous expansion chamber (60) can be assisted by manual pressurization; and
the device further comprises an air detector unit (90) downstream of the venous expansion chamber (60).

2. The method according to claim 1, wherein the manual pressurization is carried out by opening pressure sensor ports and/or disconnecting the blood inlet or blood outlet provided on the respective opposite side of the dialyser (40).

3. The method according to one of the preceding claims, wherein, starting from a point in time when in the air detector unit (90) occurrence of air is detected, a number of head revolutions of the blood pump (80) is counted, wherein a predetermined delivery volume for each revolution is associated with one head revolution as a function of a delivery constant.

4. The method according to claim 3, wherein a switch-off time of the blood pump (80) is established on the basis of a predetermined flushing amount and the counted number of head revolutions of the blood pump (80), wherein the operation of the blood pump (80) is stopped at the established switch-off time and a change from draining in the first direction to draining in the second direction is made.

5. The method according to one of the preceding claims, wherein during draining in a second direction after a predetermined time period a negative pressure is built up also on the blood side of the dialyser (40).

6. The method according to claim 5, wherein the blood-side negative pressure is measurable at the port of a venous pressure sensor (50).

7. The method according to claim 6, wherein upon detecting a predetermined blood-side negative pressure the fluid-carrying lines of the device are shut off on the water side, a pump unit in a drain for used dialysis fluid is stopped and a user is informed about the end of the blood-side draining of the device.

8. The method according to one of the preceding claims, wherein the transmembrane pressure is calculated and the draining cycle is interrupted, if the calculated transmembrane pressure exceeds a predetermined limit.

9. The method according to one of the preceding claims, wherein the membrane of the dialyser (40) is monitored by means of a blood leakage detecting unit in the drain for used dialysis fluid with respect to occurrence of a rupture.

## Revendications

1. Procédé pour la vidange d'un appareil pour le traitement extracorporel du sang, dans lequel l'appareil inclut un dialyseur (40) qui est divisé en une première chambre et une seconde chambre au moyen d'une membrane, une conduite artérielle connectée à un orifice d'entrée de sang de la première chambre, une conduite veineuse connectée à un orifice de sortie de sang de la première chambre, une conduite de liquide de dialyse connectée à un orifice d'entrée de liquide de dialyse de la seconde chambre pour un liquide de dialyse frais et une conduite de liquide de dialyse connectée à un orifice de sortie de liquide de dialyse de la seconde chambre pour un liquide de dialyse usagé, une pompe à sang (80) disposée dans la conduite artérielle et une chambre de dilatation veineuse (60) disposée dans la conduite veineuse, dans lequel
le procédé présente les étapes :
a) de connexion d'un raccord de la conduite artérielle côté patient à un raccord de la conduite veineuse côté patient ;
b) de génération d'une dépression dans la seconde chambre ;
c) d'actionnement de la pompe à sang (80) dans une première direction et de vidange des conduites artérielle et veineuse dans la première direction via la membrane et la seconde chambre, **caractérisé en ce que**
le procédé présente en outre l'étape
d) d'arrêt de la pompe à sang (80) et de vidange des conduites artérielle et veineuse dans une seconde direction opposée à la première direction via la membrane et la seconde chambre ;
l'appareil inclut en outre au moins une pompe d'ajustement de niveau qui est configurée pour préparer un ajustement de niveau au moins dans la chambre de dilatation veineuse (60), dans lequel pendant la vidange dans la première direction et pendant la vidange dans la seconde direction la pompe d'ajustement de niveau continue à fonctionner, ou pendant la vidange dans la première direction et pendant la vidange dans la seconde direction la conduite artérielle connectée à l'orifice d'entrée de sang de la première chambre et/ou la conduite veineuse connectée à l'orifice de sortie de sang de la première chambre, ainsi que la chambre de dilatation veineuse (60) peuvent être supportées manuellement par pressurisation ; et
l'appareil contient en outre un dispositif de détection d'air (90) en aval de la chambre de dilatation veineuse (60).

2. Procédé selon la revendication 1, dans lequel la pressurisation manuelle s'effectue par le biais de l'ouverture de raccords de détecteur de pression et/ou de la déconnexion de l'orifice d'entrée de sang ou de l'orifice de sortie de sang qui se trouve sur le côté opposé du dialyseur (40).

3. Procédé selon l'une quelconque des revendications précédentes, dans lequel à partir d'un moment auquel une présence d'air est détectée dans le dispositif de détection d'air (90), un nombre de rotations de tête de la pompe à sang (80) est comptabilisé, dans lequel un volume de refoulement prédéterminé par rotation est associé à une rotation de tête en fonction d'une constante de refoulement.

4. Procédé selon la revendication 3, dans lequel un moment de déconnexion de la pompe à sang (80) est établi sur la base d'une quantité de rinçage prédéterminée et du nombre comptabilisé de rotations de tête de la pompe à sang (80), dans lequel au moment de déconnexion établi le fonctionnement de la pompe à sang (80) est arrêté et un passage de la vidange dans la première direction à une vidange dans la seconde direction s'effectue.

5. Procédé selon l'une quelconque des revendications précédentes, dans lequel pendant la vidange dans une seconde direction après un laps de temps prédéterminé une dépression est également constituée sur le côté sang du dialyseur (40).

6. Procédé selon la revendication 5, dans lequel la dépression côté sang est mesurable au niveau d'un raccord d'un capteur de pression veineuse (50).

7. Procédé selon la revendication 6, dans lequel après la détection d'une dépression côté sang prédéterminée les conduites conductrices de fluide de l'appareil sont fermées côté fluide, le dispositif de pompage est arrêté lors d'une opération pour un liquide de dialyseur usagé et un opérateur est informé de la fin de la vidange de l'appareil côté sang.

8. Procédé selon l'une quelconque des revendications précédentes, dans lequel la pression transmembranaire est calculée et le processus de vidange est interrompu lorsque la pression transmembranaire calculée dépasse une valeur limite prédéterminée.

9. Procédé selon l'une quelconque des revendications précédentes, dans lequel la membrane du dialyseur (40) est surveillée au moyen d'un dispositif de détection de fuite de sang lors de l'opération pour un liquide de dialyse usagé pour déceler la présence d'une rupture.
